# EUROPEAN PATENT APPLICATION

(11) **EP 2 573 564 A1**
(43) Date of publication of application: **27.03.2013**
(21) Application number: 11182778.8
(22) Date of filing: 26.09.2011
(51) Int. Cl.: G01N 33/567, G01N 33/50

(54) **Measurement of protein modification in cells**

(71) Applicant: Philip Morris Products S.A., 2000 Neuchâtel (CH)
(72) Inventor: Stolle, Katrin, 10827 Berlin (DE); Schüller, Jutta, 51145 Cologne (DE); Lietz, Michael, 52249 Eschweiler (DE); Steffen, Yvonne, 51061 Cologne (DE); Wagner, Sandra, 52379 Langerwehe (DE); Schwenk, Tanja, 51149 Cologne (DE); Kurkowsky, Birgit, 51149 Cologne (DE); Weisensee, Dirk, 51149 Cologne (DE)
(74) Representative: Vossius & Partner

(57) **Abstract**

A highly sensitive and specific method is provided for determining the presence of oxidative stress in cells or tissues of a subject, which method is based on simultaneous detection of both, the protein carbonyl and 3-nitrotyrosine biomarker, in a single assay format in intact cells. This method combines the advantages of an ELISA (sensitivity) method and digital image analysis of cellular fluorescence signals and allows spatial and temporal resolution of oxidative stress responses on a single cell level.

## Description

The present invention provides a highly sensitive and specific method for determining the presence of oxidative stress in cells or tissues of a subject, which method is based on simultaneous detection of both, the protein carbonyl and 3-nitrotyrosine biomarker, in a single assay format in intact cells. This method combines the advantages of an ELISA (sensitivity) method and digital image analysis of cellular fluorescence signals and allows spatial and temporal resolution of oxidative stress responses on a single cell level.

Protein carbonyls and 3-nitrotyrosine (as an indirect marker of peroxynitrite) are considered as markers of oxidative stress in cells which can be caused by cigarette smoke. The usage of protein carbonyls (CO) as biomarkers of oxidative stress has shown several advantages in comparison to the measurement of other oxidation products. Carbonylated proteins are formed early after exposure of cells or tissues to oxidative stress and are relatively stable, thus they can be reliably detected in samples. Most assays for detection of protein CO moieties involve the derivatisation of the carbonyl group with 2,4-dinitrophenylhydrazine (DNPH), which leads to the formation of a stable dinitrophenyl (DNP) hydrazone products. These can be detected in cell lysates by various means, such as enzyme-linked immunosorbent assay (ELISA) or electrophoresis followed by Western blot immunoassay. So far it is not possible to detect and quantify protein carbonyls in single cells. Beside oxidation reactions in proteins cigarette smoke may trigger and modify biologic membranes and proteins via nitration of proteins.

Peroxynitrite is formed through the reaction between nitric oxide and superoxide in inflamed tissues. Peroxynitrite can interact with nucleic acids, causing DNA strand breakage, modify proteins and react with guanine residues with the formation of 8-nitroguanine, thus contributing to the multistage process of e.g. atheriosclerosis, carcinogenesis. Through the formation of peroxynitrite, nitration of tyrosine residues of proteins occurs. Nitration of tyrosine residues of proteins leads to the production of 3-nitrotyrosine, which may be considered as a marker of nitric oxide-dependent oxidative damage and can be detected by dot blotting.

Buss et al (1997) and Shacter et al (1994) describe conventional assays for protein carbonyls, which are based on ELISA and Western blot techniques in cell lysates. These assays involve derivatisation of the carbonyl group with 2,4-dinitrophenylhydrazine (DNPH), which leads to the formation of a stable dinitrophenyl hydrazone (DNP) product. The reaction takes place in the presence of concentrated HCl or orthophosphoric acid. Therefore the assays can only be performed in cell lysates but not in intact cells because of the high acid concentrations, which destroy the complete cell scaffold. These assays further require high amounts of cellular proteins and are labor intensive.

Qu et al (2003) describes novel competitive ELISA for both free and protein-bound nitrotyrosine.

US 2004132111 describes two independent immunological assays for analysing protein carbonyl and protein nitro-tyrosine formation in homogenized tissue samples by determining the amount of DNPH derivatized protein carbonyls and nitro-tyrosines in the homogenized material.

Presently available qualitative methods like, e.g. the Oxyblot (Cayman) technique increases selectivity, but have not been developed as a quantitative assay (D'Sa et al (2007)). Currently there are no assays available combining the measurement of protein carbonyl and 3-nitrotyrosine moieties in one assay in intact cells.

There was therefore a need for improved assay formats for analyzing these two important markers of oxidative stress using one single test system that allows quantitative as well as qualitative measurements in intact cells.

This need was complied with in the present invention by providing the methods as now claimed. The claimed methods have several benefits and advantages in that they are highly sensitive and specific and allow simultaneous detection of both, the protein carbonyl and 3-nitrotyrosine biomarker, in a single assay format in intact cells. Further, automated digital image analysis can be used to quantify the fluorescence signals for both biomarkers in individual cells in a single step.

In particular, the present invention provides a method for determining the presence of oxidative stress in cells or tissues of a subject comprising the steps of: (i) providing a cell or tissue sample from a subject; (ii) incubating the cell or tissue sample with 2,4-dinitrophenylhydrazine (DNPH), which is particularly provided as a dilute acid solution, to convert protein carbonyl groups of proteins present in the cell or tissue sample to the corresponding 2,4-dinitrophenylhydrazones; (iii) contacting the DNPH treated cell or tissue sample with anti-DNPH antibody and maintaining that contact for a time period sufficient to form an immunocomplex between the derivatized protein carbonyl groups of said proteins and the anti-DNPH antibody; (iv) contacting the same cell or tissue sample with anti-nitrotyrosin antibody for a time period sufficient to form an immunocomplex between the derivatized tyrosine residues on proteins present in the cell or tissue sample and the anti-nitrotyrosin antibody; and (v) determining the amount of anti-DNPH antibody- and anti-nitrotyrosin antibody-immunocomplex in the cell or tissue sample and comparing that amount to the amount of immunocomplex present in a standard sample, wherein an amount greater than that present in the standard sample in excess of experimental error is indicative for the presence of oxidative stress in the subject.

In a specific aspect of the present invention, the cell or tissue sample is a matter of intact cells or tissues, which, in another aspect of the invention, are intact cells or tissues bound or attached to a solid support.

In one aspect of the invention, said cell or tissue samples are obtained from a subject, which has a history of smoking.

The solid support used for supporting the cells or tissues to be analyzed, may be made of any material selected from the group consisting of polymers, glass, metals or silicon; said material being able to fix or bind directly or be covered by a layer able to fix or bind the cells or tissues to be analysed. The forms of the supports are preferentially, but not limited to, glass slides, coverslips, plastic supports, multi-well plates of 96, 384 or 1536 wells, micro-arrays with planar surfaces or with cavities. Also contemplated for use in the present invention are microbeads, which may be assayed in fluorescene-activated cell sorter (FACS) or used for facilitating their washing through their magnetic properties.

In one embodiment, the amount of immunocomplex present in the cell or tissue sample is determined within the method according to the preceding embodiments by binding the immunocomplex to a second antibody and measuring the amount of bound secondary antibody.

In one embodiment, the present invention provides a method according to any one of the preceding embodiments, wherein the first or second antibody, or the first and second antibody, is labeled with a fluorescent tag such as, for example, a fluorescein isothiocyanate (FITC) or a phycoerythrin (PE) fluorescent tag, a radioactive molecule, or an indicator enzyme such as, for example, horseradish peroxidase.

Accordingly, the amount of immunocomplex present in the cell or tissue sample may be determined by fluorescence spectroscopy, ultra-violet spectroscopy, or by radiography.

In a specific aspect of the invention, the method of any one of the preceding embodiments comprises the additional step of determining the qualitative distribution of the immunocomplex within the cell or tissue.

In another embodiment of the invention, a method is provided for determining spatial and temporal distribution of oxidative stress responses in a cell or tissue sample, comprising the steps of (i) providing a cell or tissue sample from a subject; (ii) incubating the cell or tissue sample with 2,4-dinitrophenylhydrazine (DNPH), which is particularly provided as a dilute acid solution, to convert protein carbonyl groups of proteins present in the cell or tissue sample to the corresponding 2,4-dinitrophenylhydrazones; (iii) contacting the DNPH treated cell or tissue sample with anti-DNPH antibody and maintaining that contact for a time period sufficient to form an immunocomplex between the derivatized protein carbonyl groups of said proteins and the anti-DNPH antibody; (iv) contacting the same cell or tissue sample with anti-nitrotyrosin antibody for a time period sufficient to form an immunocomplex between the derivatized tyrosine residues on proteins present in the cell or tissue sample and the anti-nitrotyrosin antibody; and (v) determining the spatial and temporal distribution of anti-DNPH antibody- and anti-nitrotyrosin antibody-immunocomplex in the cell or tissue sample.

In still another embodiment, the invention provides a method for quantifying oxidative stress responses in a cell or tissue sample, comprising the steps of: (i) providing a cell or tissue sample from a subject; (ii) incubating the cell or tissue sample with 2,4-dinitrophenylhydrazine (DNPH), which is particularly provided as a dilute acid solution, to convert protein carbonyl groups of proteins present in the cell or tissue sample to the corresponding 2,4-dinitrophenylhydrazones; (iii) contacting the DNPH treated cell or tissue sample with anti-DNPH antibody and maintaining that contact for a time period sufficient to form an immunocomplex between the derivatized protein carbonyl groups of said proteins and the anti-DNPH antibody; (iv) contacting the same cell or tissue sample with anti-nitrotyrosin antibody for a time period sufficient to form an immunocomplex between the derivatized tyrosine residues on proteins present in the cell or tissue sample and the anti-nitrotyrosin antibody; and (v) determining the amount of anti-DNPH antibody- and anti-nitrotyrosin antibody-immunocomplex in the cell or tissue sample.

In one aspect of the invention, a method is provided for monitoring the progress of a treatment for reducing oxidative stress in an individual, said method comprising quantifying, at different time points during the treatment, the oxidative stress response in a cell or tissue sample taken from said individual applying the method according to the present invention and as described herein.

Another aspect to the invention relates to a method for screening compounds for their potential for inducing oxidative stress in a subject, comprising the steps of: (i) providing a cell or tissue sample from a subject; (ii) contacting said cell or tissue sample with a test compound, (iii) incubating the treated cell or tissue sample with 2,4-dinitrophenylhydrazine (DNPH), which is particularly provided as a dilute acid solution, to convert protein carbonyl groups of proteins present in the cell or tissue sample to the corresponding 2,4-dinitrophenylhydrazones; (iv) contacting the DNPH treated cell or tissue sample with anti-DNPH antibody and maintaining that contact for a time period sufficient to form an immunocomplex between the derivatized protein carbonyl groups of said proteins and the anti-DNPH antibody; (v) contacting the same cell or tissue sample with anti-nitrotyrosin antibody for a time period sufficient to form an immunocomplex between the derivatized tyrosine residues on proteins present in the cell or tissue sample and the anti-nitrotyrosin antibody; and (vi) determining the amount of anti-DNPH antibody- and anti-nitrotyrosin antibody-immunocomplex in the cell or tissue sample treated with the test compound and comparing that amount to the amount of immunocomplex present in an untreated sample, wherein an amount greater than that present in the untreated sample in excess of experimental error is indicative for the oxidative stress-inducing potential of the test compound.

Test compounds for use in this method may be obtained by fractionating the constituents of an aerosol generated by heating or combusting a tobacco product, but particularly the constituents of cigarette smoke, particularly the constituents of the total particulate matter or the gas vapour phase of cigarette smoke.

Thus, in one embodiment, the present invention provides a method of determining potential of the constituents of an aerosol generated by heating or combusting a tobacco product, but particularly of the constituents of cigarette smoke, particularly of the constituents of the total particulate matter or the gas vapour phase of cigarette smoke, for inducing oxidative stress in a subject, which method comprises the steps of (i) providing a cell or tissue sample from a subject; (ii) contacting said cell or tissue sample with a test compound obtained by fractionating the constituents of an aerosol generated by heating or combusting a tobacco product, but particularly the constituents of cigarette smoke, particularly the constituents of the total particulate matter or the gas vapour phase of cigarette smoke, (iii) incubating the treated cell or tissue sample with 2,4-dinitrophenylhydrazine (DNPH), which is particularly provided as a dilute acid solution, to convert protein carbonyl groups of proteins present in the cell or tissue sample to the corresponding 2,4-dinitrophenylhydrazones; (iv) contacting the DNPH treated cell or tissue sample with anti-DNPH antibody and maintaining that contact for a time period sufficient to form an immunocomplex between the derivatized protein carbonyl groups of said proteins and the anti-DNPH antibody; (v) contacting the same cell or tissue sample with anti-nitrotyrosin antibody for a time period sufficient to form an immunocomplex between the derivatized tyrosine residues on proteins present in the cell or tissue sample and the anti-nitrotyrosin antibody; and (vi) determining the amount of anti-DNPH antibody- and anti-nitrotyrosin antibody-immunocomplex in the cell or tissue sample treated with the test compound and comparing that amount to the amount of immunocomplex present in an untreated sample, wherein an amount greater than that present in the untreated sample in excess of experimental error is indicative for the oxidative stress-inducing potential of the test compound.

The foregoing description will be more fully understood with the reference to the following Examples. Such Examples are, however, exemplary methods of practicing the present invention and are not intended to limit the scope of the invention.

### EXAMPLES

### 1. Detection of Protein Carbonyl:

Human Umbilical Vein Endothelial Cells (HUVEC) or human endothelial Ea.hy 926 cells were seeded in 96-well plates and treated with cigarette smoke fractions for 24 hours. After the incubation period cells were fixed with 2.5% and 10% paraformaldehyde, respectively, in PBS for 30 min at room temperature. After removal of the paraformaldehyde solution, cells were incubated for 1 h at room temperature with DNPH (300 mg DNPH per 100 ml 96% ethanol containing 1.5% pure sulfuric acid) in order to convert protein carbonyl groups to the corresponding 2,4-dinitrophenylhydrazones detectable with an anti-DNPH antibody. The DNPH solution was filtered with a 0.2-pm filter before use. To remove residual DNPH, cells were first extensively washed with PBS (at least 3 times) and then incubated for 60 min at RT in blocking solution (3 % FCS (fetal calf serum) and 0.3% Triton X-100). The primary DNP adduct-specific goat antibody (Sigma) was applied in a dilution of 1:100 in PBS for 1 h at room temperature, and after 1 time washing with PBS the cells were treated with the secondary goat Alexa Fluor 546 nm antibody (Invitrogen) in a dilution of 1:500 in PBS for 1 h at room temperature. After removal of the secondary antibody the cells were washed 3 times with PBS.

### 2. Detection of Nitro-Tyrosine:

Thereafter the same cells were incubated with mouse monoclonal anti-nitrotyrosine antibody (Sigma) or rabbit anti-nitrotyrosine antibody at a 1:50 dilution overnight at 4°C. After the cells had been washed 3 times in PBS, a secondary antibody, Alexa Fluor 488 (Invitrogen), was applied in a dilution of 1:500 in PBS and incubated for 1 h at room temperature followed by nuclear counterstaining with Hoechst dye (1 mg/ml 1: 100). The plate was measure with with the Arrayscan reader (Cellomics) and the amount of protein carbonyls and 3-Nitrotyrosine in the cells was quantified with the NUCTrans V3 ox. Stressl Hoechst/TRIG or Hoechst/FITC program. In parallel, fluorescence images (e.g. single and overlay images) for qualitative analysis were taken.

### 3. Result:

The here described method is sensitive and also highly specific and combines the advantages of an ELISA (sensitivity) method and digital image analysis of cellular fluorescence signals. Simultaneous determination of protein carbonyls and 3-nitrotyrosine in intact cells allows spatial and temporal resolution of oxidative stress responses on a single cell level.

### LIST OF REFERENCES

H. D'Sa, B. Hettinga, A. Safdar, M. A. Tarnopoisky, M. J. Hamadeh, S. Raha The use of DNPHderivatized protein carbonyls as a marker of oxidative stress in mouse heart and liver The FASEB Journal. (2007) 21:Ib478
E. Shacter, J. A. Williams, M. Lim and R. L. Levine, Differential susceptibility of plasma proteins to oxidative modification: Examination by Western blot immunoassay. Free Radic. Biol. Med. 17 (1994), pp. 429-437
H. Buss, T.P. Chan, K.B. Sluis, N.M. Domigan and C.C. Winterbourn, Protein carbonyl measurement by a sensitive ELISA method, Free Radic. Biol. Med. 23 (1997), pp. 361-366.
Qu LN, Yang TB, Yuan YH, Zhong P, Yang B, Zhao H.A novel competitive ELISA for both free and protein-bound nitrotyrosine Hybrid Hybridomics.22 (2003) pp:401-406.

## Claims

1. A method for determining the presence of oxidative stress in cells or tissues of a subject comprising the steps of: (i) providing a cell or tissue sample from a subject; (ii) incubating the cell or tissue sample with 2,4-dinitrophenylhydrazine (DNPH) to convert protein carbonyl groups of proteins present in the cell or tissue sample to the corresponding 2,4-dinitrophenyfhydrazones; (iii) contacting the DNPH treated cell or tissue sample with anti-DNPH antibody for a time period sufficient to form an immunocomplex between the derivatized protein carbonyl groups of said proteins and the anti-DNPH antibody; (iv) contacting the same cell or tissue sample with anti-nitrotyrosine antibody for a time period sufficient to form an immunocomplex between the derivatized tyrosine residues on proteins present in the cell or tissue sample and the anti-nitrotyrosine antibody; and (v) determining the amount of anti-DNPH antibody and anti-nitrotyrosine antibody-immunocomplex in the cell or tissue sample and comparing that amount to the amount of immunocomplex present in a standard sample, wherein an amount greater than that present in the standard sample in excess of experimental error is indicative for the presence of oxidative stress in the subject.

2. The method of claim 1, wherein, in an additional step, the qualitative distribution of the immunocomplex within the cell or tissue is determined.

3. A method for determining spatial and temporal distribution of oxidative stress responses in a cell or tissue sample, comprising the steps of: (i) providing a cell or tissue sample from a subject; (ii) incubating the cell or tissue sample with 2,4-dinitrophenylhydrazine (DNPH) to convert protein carbonyl groups of proteins present in the cell or tissue sample to the corresponding 2,4-dinitrophenylhydrazones; (iii) contacting the DNPH treated cell or tissue sample with anti-DNPH antibody for a time period sufficient to form an immunocomplex between the derivatized protein carbonyl groups of said proteins and the anti-DNPH antibody; (iv) contacting the same cell or tissue sample with anti-nitrotyrosine antibody for a time period sufficient to form an immunocomplex between the derivatized tyrosine residues on proteins present in the cell or tissue sample and the anti-nitrotyrosine antibody; and (v) determining the spatial and temporal distribution of anti-DNPH antibody and anti-nitrotyrosine antibody-immunocomplex in the cell or tissue sample.

4. A method for quantifying oxidative stress responses in a cell or tissue sample, comprising the steps of: (i) providing a cell or tissue sample from a subject; (ii) incubating the cell or tissue sample with 2,4-dinitrophenylhydrazine (DNPH) to convert protein carbonyl groups of proteins present in the cell or tissue sample to the corresponding 2,4-dinitrophenylhydrazones; (iii) contacting the DNPH treated cell or tissue sample with anti-DNPH antibody for a time period sufficient to form an immunocomplex between the derivatized protein carbonyl groups of said proteins and the anti-DNPH antibody; (iv) contacting the same cell or tissue sample with anti-nitrotyrosine antibody for a time period sufficient to form an immunocomplex between the derivatized tyrosine residues on proteins present in the cell or tissue sample and the anti-nitrotyrosine antibody; and (v) determining the amount of anti-DNPH antibody-immunocomplex and anti-nitrotyrosine antibody-immunocomplex in the cell or tissue sample.

5. A method for monitoring the progress of a treatment for reducing oxidative stress in an individual, said method comprising quantifying the oxidative stress response in a cell or tissue sample obtained from said individual at different time points during the treatment, by applying the method according to claim 4.

6. A method for determining the potential of a compound for inducing oxidative stress in a subject, comprising the steps of: (i) providing a cell or tissue sample from a subject; (ii) contacting said cell or tissue sample with a test compound, (iii) incubating the treated cell or tissue sample with 2,4-dinitrophenylhydrazine (DNPH) to convert protein carbonyl groups of proteins present in the cell or tissue sample to the corresponding 2,4-dinitrophenylhydrazones; (iv) contacting the DNPH treated cell or tissue sample with anti-DNPH antibody for a time period sufficient to form an immunocomplex between the derivatized protein carbonyl groups of said proteins and the anti-DNPH antibody; (v) contacting the same cell or tissue sample with anti-nitrotyrosine antibody for a time period sufficient to form an immunocomplex between the derivatized tyrosine residues on proteins present in the cell or tissue sample and the anti-nitrotyrosine antibody; and (vi) determining the amount of anti-DNPH antibody- and anti-nitrotyrosine antibody-immunocomplex in the cell or tissue sample treated with the test compound and comparing that amount to the amount of immunocomplex present in an untreated sample, wherein an amount greater than that present in the untreated sample in excess of experimental error is indicative for the oxidative stress-inducing potential of the test compound.

7. The method of claim 6, wherein the test compound is a constituent of cigarette smoke.

8. The method of any one of the preceding claims, wherein the determination of protein carbonyl and nitro-tyrosine is carried out in intact cells or tissues.

9. The method of any one of the preceding claims, wherein the cell or tissue test sample is from a smoker, the reference sample from a non-smoker.

10. The method of any one of the preceding claims, wherein said cell or tissue sample is bound or attached to a solid support.

11. The method of claim 8, wherein said solid support is provided in form of glass slides, coverslips, plastic supports, multi-well plates of 96, 384 or 1536 wells, micro-arrays with planar surfaces or with cavities, or microbeads.

12. The method of any one of the preceding claims, wherein the amount of immunocomplex present in the cell or tissue sample is determined by binding the immunocomplex to a second antibody and measuring the amount of bound secondary antibody.

13. The method of any one of the preceding claims, wherein the first or second antibody, or both the first and second antibody, are labeled with
(i) a fluorescent tag; or
(ii) an indicator enzyme, or
(iii) a radioactive molecule.

14. The method of claim 13, wherein said indicator enzyme is horseradish peroxidase.

15. The method of any one of the preceding claims, wherein the amount of immunocomplex present in the cell or tissue sample is determined by
(i) fluorescence spectroscopy; or
(ii) ultra-violet spectroscopy, or
(iii) radiography
